# EUROPEAN PATENT SPECIFICATION

(11) **EP 0 690 733 B1**
(45) Date of publication and mention of the grant of the patent: **16.06.1999**
(21) Application number: 95908403.9
(22) Date of filing: 23.01.1995
(51) Int. Cl.: A61M 15/00

(54) **AN INHALATION CHAMBER FOR CHILDREN FOR USE IN CONJUNCTION WITH A METERED DOSE INHALER**
INHALATIONSKAMMER FÜR KINDER ZUM GEBRAUCH MIT EINER VORRICHTUNG ZUR DOSIERTEN INHALIERUNG
CHAMBRE D'INHALATION PREVUE POUR ETRE UTILISEE AVEC UN INHALATEUR DOSEUR POUR ENFANT

(30) Priority: 27.01.1994 SE 9400257
(43) Date of publication of application: 10.01.1996
(73) Proprietor: Astra Aktiebolag, 151 85 Södertälje (SE)
(72) Inventor: BERG, Elna, Birgitta, S-240 10 Dalby (SE); NILSSON, Hans, Jörgen, S-224 66 Lund (SE)
(86) International application number: PCT/SE95/00058
(87) International publication number: WO 95/20414

(56) References cited:
- EP-A- 0 385 212
- WO-A-93/16747
- GB-A- 2 110 543
- SE-B- 411 705

## Description

The present invention relates to a spacer for use with a metered dose inhaler (MDI).

In order to alleviate the problems of high oral deposition and the coordination difficulties associated with metered dose inhalers, spacers have been developed for use therewith. One end of a spacer is connected to a metered dose inhaler and the other end is connected to or provided with a mouthpiece or face mask through which a patient inhales. When the metered dose inhaler is actuated, gaseous propellant entraining a dose of drug is sprayed into the spacer, thereby providing a cloud of small particles of respirable size in the spacer for a certain period of time, during which time large particles, that is, particles which would normally would deposit orally, settle and deposit in the spacer. The aerosol cloud of small particles can be inhaled effortlessly by a patient.

Prior art spacers of this kind are, however, exclusively designed for use with older children and adults. The volume of such spacers is normally in the range of from 0.5 to 2 litres and such spacers are principally made of a polymeric material, for instance, polycarbonate. Such spacers are often adapted for use in younger children. However, the reproducibility and age-dependency of the dose delivery when used for the treatment of children is important, and as yet has not been ascertained. Theoretical models have predicted an increased lung deposition in small children due to the smaller airways and a greater ventilation per kilogram, but in vivo documentation is sparse. Most of the available in vivo documentation for the dose delivery of prior art spacers relates to the clinical response in wheezy children on inhalation of a β₂-agonist. In these studies the drug has been administered in doses considerably above the minimum effective dose and the response is therefore not critically dependent on reproducible delivery. In addition, the results from studies with these drugs cannot be extended to the generation of steroid aerosol systems which exhibit other micronizing and solubility characteristics.

There is therefore a need for a spacer which is adapted to the treatment of young children, particularly for the administration of steroids for which the demands on reproducibility and dose accuracy are stricter and a high utilisation of which is desirable.

The volume of the spacer is critical since it is emptied in an exponential manner. The inspiratory volume required to inhale entirely the aerosol cloud form the spacer will be several times the volume of the spacer, but the fall out of particles from the aerosol cloud limits the time available for inhalation. Since the inspirational capability of small children is less than the inspirational capability of adults, ideally, the volume of the spacer should be reduced, for instance, to the order of a few tidal breaths of an infant so as to reduce the time required for administration. Whilst a spacer of reduced volume will contain an increased concentration of drug and require less time to empty, the fraction of airborne particles will be reduced due to the deposition, settling and agglomeration of particles. This effect will be pronounced if the spacer is made of a polymeric material, such as polycarbonate, which may be charged electrostatically.

It is thus an aim of the present invention to provide an improved spacer for use with small children.

Accordingly, the present invention provides a spacer for use with a metered dose inhaler, comprising a body which defines a chamber, wherein the body has a generally oblong shape which is rotationally symmetrical about a longitudinal axis and an opening at each end, one of the openings being adapted for connection to a metered dose inhaler; characterized in that the body is composed of metal having a surface resistivity less than 1 Ohm and in that the chamber has a total volume of between 50 and 400 ml.

Preferably, the body is composed of stainless steel.

A preferred embodiment of the present invention will now be described hereinbelow by way of example only with reference to the accompanying drawings, in which:

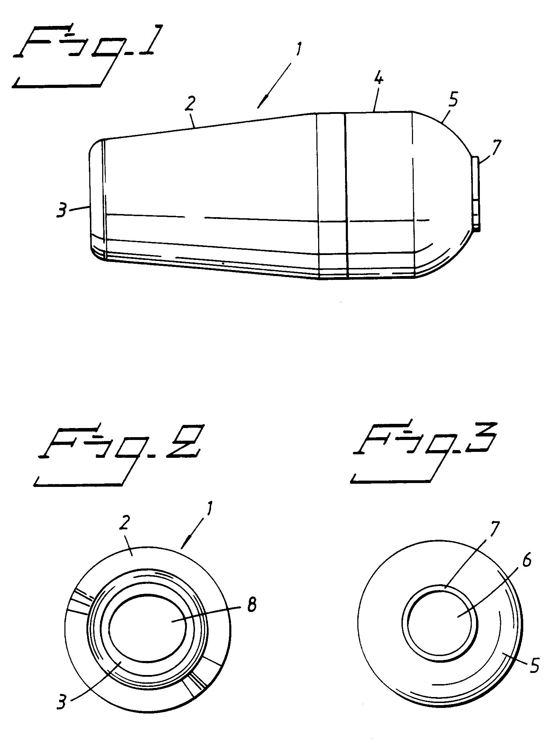

Figure 1 illustrates a side view of a spacer in accordance with a preferred embodiment of the present invention;
Figure 2 illustrates the end of the spacer of Figure 1 to which a metered dose inhaler is connected; and
Figure 3 illustrates the end of the spacer of Figure 1 to which a mouthpiece or face mask is connected.

The spacer comprises an oblong body 1 which has a rotationally symmetrical circular section. The body 1 has a slightly tapering frusto-conical part 2, the small end of which is rounded and defines one end 3 of the body 1, a central cylindrical part 4 and a substantially hemi-spherical part 5 which defines the other end of the body 1. The hemi-spherical part 5 includes a central opening 6 having a projecting, circumferential flange 7 for the connection of a mouthpiece or face mask. A two-way valve may be connected between the spacer and the mouthpiece or face mask. The frusto-conical part 2 includes a central opening 8, in this embodiment elliptical in shape, adapted to fit the mouthpiece of a metered dose inhaler.

The spacer has an overall length of about 130 mm, with the length of the frusto-conical part 2 being about 80 mm, the diameter of the one end 3 being about 40 mm (the rounded section of the frusto-conical part 2 being disregarded) and the diameter of the cylindrical part 4 being about 55 mm. In this embodiment the volume of the spacer is 220 ml. The spacer of this embodiment having such dimensions is suited to most small children, although other dimensions are conceivable. The length of the spacer is such that particles of respirable size are not sprayed onto opposing surfaces of the body 1 of the spacer when the metered dose inhaler is actuated.

In this embodiment the spacer is made of stainless steel, which material has a surface resistivity of less than 1 Ohm and is sufficiently low as to minimize the electrostatic attraction of particles of respirable size to surfaces of the body 1 of the spacer. In forming the spacer of stainless steel the spacer is also very robust which makes it particularly suitable for use with small children.

In use, the mouthpiece of a metered dose inhaler is fitted into the opening 8 in the frusto-conical part 2 and a mouthpiece or face mask incorporating a two-way valve is fitted into the opening 6 in the hemi-spherical part 5. The two-way valve allows air to be inhaled through the spacer from the metered dose inhaler to the mouthpiece or face mask, but prevents air from being exhaled into the spacer. In this way, air flow through the spacer is rectified.

By forming the spacer of a material of low resitivity and of small volume, the reduction in the volume of air which has to be inhaled enables the delivery of a reasonably reproducible and accurate dose of drug to small children. In addition, the spacer enables the utilisation of a larger fraction of drug than possible in standard prior art spacers.

## Claims

1. A spacer for use with a metered dose inhaler (MDI), comprising a body (1) which defines a chamber, wherein the body (1) has a generally oblong shape which is rotationally symmetrical about a longitudinal axis and an opening (6, 8) at each end, one of the openings (8) being adapted for connection to a metered dose inhaler; characterized in that the body (1) is composed of metal having a surface resistivity less than 1 Ohm and in that the chamber has a total volume of between 50 and 400 ml.

2. The spacer according to claim 1, wherein the body (1) is composed of stainless steel.

## Patentansprüche

1. Abstandsstück zur Verwendung bei einer Vorrichtung zur dosierten Inhalierung, mit einem eine Kammer definierenden Körper (1), der eine allgemein längliche Form, die an einer Längsachse rotationssymmetrisch ist, und an jedem Ende eine Öffnung (6, 8) aufweist, wobei eine der Öffnungen (8) zur Verbindung mit einer Vorrichtung zur dosierten Inhalierung ausgeführt ist; dadurch gekennzeichnet, daß der Körper (1) aus Metall mit einem spezifischen Oberflächenwiderstand von unter 1 Ohm besteht und daß die Kammer ein Gesamtvolumen zwischen 50 und 400 ml aufweist.

2. Abstandsstück nach Anspruch 1, bei dem der Körper (1) aus rostfreiem Stahl besteht.

## Revendications

1. Pièce d'écartement destinée à être utilisée dans un inhalateur doseur (MDI), comprenant un corps (1) qui définit une chambre, dans laquelle le corps (1) présente une forme généralement oblongue qui est symétrique en rotation autour d'un axe longitudinal, et une ouverture (6, 8) à chaque extrémité, l'une des ouvertures (8) étant destinée à être raccordée à un inhalateur doseur, caractérisée en ce que le corps (1) est composé d'un métal présentant une résistivité de surface inférieure à 1 ohm et en ce que la chambre présente un volume total compris entre 50 et 400 ml.

2. Pièce d'écartement suivant la revendication 1, dans laquelle le corps (1) est composé d'acier inoxydable.
